# EUROPEAN PATENT SPECIFICATION

(11) **EP 4 034 126 B1**
(45) Date of publication and mention of the grant of the patent: **04.11.2026**
(21) Application number: 20868726.9
(22) Date of filing: 05.08.2020
(51) Int. Cl.: A61K 9/00, A61K 9/20, A61K 47/10, A61K 47/12, A61K 47/22, A61K 31/519

(54) **PHARMACEUTICAL FORMULATIONS COMPRISING SAPROPTERIN DIHYDROCHLORIDE AND AT LEAST ONE ANTIOXIDANT**
PHARMAZEUTISCHE FORMULIERUNGEN, DIE SAPROPTERIN-DIHYDROCHLORID UND MINDESTENS EIN ANTIOXIDANS ENTHALTEN
FORMULATIONS PHARMACEUTIQUES COMPRENANT DU DICHLORHYDRATE DE SAPROPTÉRINE ET AU MOINS UN ANTIOXYDANT

(30) Priority: 23.09.2019 TR 201914420
(43) Date of publication of application: 03.08.2022
(73) Proprietor: Sanovel Ilac Sanayi ve Ticaret A.S., Sisli - Istanbul (TR)
(72) Inventor: PALANTOKEN, Arzu, 34460 Sariyer/Istanbul (TR); TURKOGLU, Damla, 34460 Sariyer/Istanbul (TR)
(74) Representative: Genç Ilhan, Oznur
(86) International application number: PCT/TR2020/050690
(87) International publication number: WO 2021/061067

(56) References cited:
- WO-A1-2008/089148
- WO-A2-2006/055511
- CN-A- 104 257 623
- US-A1- 2013 108 694
- US-A1- 2013 336 945

## Description

### Field of the invention

The present invention relates to pharmaceutical formulations comprising sapropterin dihydrochloride and at least one antioxidant.

### Background of the invention

Phenylketonuria (PKU) is a rare condition where babies are born unable to break down an amino acid called phenylalanine. This causes phenylalanine to build up.

When phenylalanine levels get too high, it can cause damage to the brain. This can lead to intellectual and developmental disabilities.

Phenylalanine is found in foods that contain protein. PKU is manageable, mostly through your diet. The key to living with PKU successfully is finding it early. That is why all babies in the United States are screened for the disease at birth.

PKU is an inherited disease. This means it is passed down through the genes of the mother and father. It is caused by mutations in the gene that helps make an enzyme called phenylalanine hydroxylase. This enzyme helps break down phenylalanine. When this gene doesn't work right, the body can't break down phenylalanine. It starts to build up and causes damage to nerve cells in the brain.

Initially in newborns there are no symptoms. But within a few months of birth, depending on the severity of the disease, symptoms begin to show. These include:
- Smaller than normal head size (called microcephaly)
- Hyperactivity
- A musty or mouse-like odor in urine, breath, or skin
- Lighter skin, hair, and eyes than their siblings
- Skin disorders such as eczema
- Jerking movements of the arms or legs
- Tremors
- Seizures
- Delayed development
- Behavioral problems
- Psychiatric disorders
- Permanent intellectual disability

If PKU is not controlled through changes in diet, it can cause severe intellectual and developmental disability.

The U.S. Food and Drug Administration has approved the medicine sapropterin dihydrochloride (Kuvan^{®}) for the treatment of PKU. Kuvan can help the body break down phenylalanine.

Sapropterin dihydrochloride (Kuvan^{®} [BioMarin, CA, USA]) is the first and only registered synthetic form of the naturally occurring enzyme cofactor, BH4 (5,6,7,8-tetrahydrobiopterin) Sapropterin is an orally active, synthetic dihydrochloride salt formulation of the Biologically active 6R-diastereoisomer of BH4 (5,6,7,8-tetrahydrobiopterin) Although PKU is characterized by a defect in the PAH (Phenylalanine hydroxylase) enzyme, residual enzymatic activity may be present in some patients. Thus, sapropterin may act like a chemical chaperone to promote the normal metabolism of Phe(Phenylalanine) and lower its concentration in the blood in a subset of patients who are BH4 responsive. Sapropterin may be used to assist in the control of Phe concentrations. It provides the opportunity for patients who respond to BH4 to adjust their diet, thereby allowing a greater intake of Phe or even coming off of their Phe-restrictive diet. The overall frequency of BH4 responsiveness across Europe is estimated to be 55-62%, based on projections made using genetic allelic data for BH4 responsiveness, although responsiveness can only be determined by a response test.

The chemical name of sapropterin dihydrochloride is (6R)-2-amino-6-[(1R,2S)-1 ,2-dihydroxypropyl]-5,6,7,8-tetrahydro-4(1H)-pteridinone dihydrochloride and the molecular formula is C9H15N5O3·2HCl with a molecular weight of 314.17. Sapropterin dihydrochloride has the following structural formula:

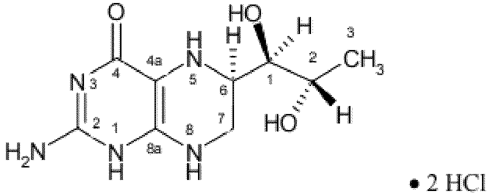

The patent US7727987B2 of Merck discloses crystal forms of (6R)-L-erythro-tetrahydrobiopterin dihydrochloride and hydrates and solvates thereof. Also relates to compositions comprising selected and stable crystal forms of (6R)-L-erythro-tetrahydrobiopterin dihydrochloride or a hydrate thereof and a pharmaceutically acceptable carrier.

The patent EP1757293B1 of Daiichi Sankyo discloses an effective sapropterin hydrochloride preparation that is highly stable during storage and can administer sapropterin hydrochloride, an effective therapeutic agent for the treatment of tetrahydrobiopterin-responsive hyperphenylalaninemia, to patients of a wide range of ages, ranging from infants to adults, in a single preparation

The patent application EP1845952A2 of BioMarin Pharmaceutical discloses a stable solid formulation of tetrahydrobiopterin, or a precursor or derivative or analog thereof, that maintains its stability for an extended period of time. Compositions of the invention may comprise a stable, crystalline form of BH4 that is stable at room temperature for more than 8 hours and a pharmaceutically acceptable carrier, diluent or excipient. Exemplary stable tablets of the invention have been prepared using a dry tableting process and have been shown to have a shelf-life of at least 6 to 9 months at room temperature.

The patent application EP2680848A1 of Dipharma discloses stable pharmaceutical compositions comprising tetrahydrobiopterin and at least one stabilizing agent. Particularly the present invention relates to stable compositions of sapropterin dihydrochloride. Antioxidants that stabilize tetrahydrobiopterin; with the weight ratio of the antioxidant to active ranging from 0.2 - 1.5.

The patent application CN104257623A patent of Guangdong Zhongsheng pharmaceutical company discloses an effervescent tablet containing sapropterin dihydrochloride.

WO2006055511 and US2013336945 discloses sapropterin dihydrochloride compositions comprising antioxidants.

Sapropterin dihydrochloride exhibits polymorphism and many crystalline forms have been identified; among all the polymorphic forms, Form B was identified to be thermodynamically stable crystalline anhydrate form. Polymorph form B is a very stable crystalline form, that can be easily filtered off, dried and ground to particle sizes desired for pharmaceutical formulations. These outstanding properties renders polymorph form B especially feasible for pharmaceutical application.

Sapropterin dihydrochloride is currently available as oral soluble tablets of 100mg and 100mg-500mg powder packets under the brand name Kuvan^{™}. It is marketed by BioMarin in the US and Merck Serono in Europe. Kuvan^{™} has been designated as an orphan medication since hyperphenylalaninemia is a rare disease. Kuvan^{™} is indicated to reduce blood phenylalanine levels in patients with hyperphenylalaninemia due to tetrahydrobiopterin responsive phenylketonuria. It is to be used in conjunction with phenylalanine restricted diet. In patients with phenylketonuria the role of sapropterin dihydrochloride is to enable endogenous phenylalanine hydroxylase activity and to partially restore oxidative metabolism of phenylalanine, resulting in decreased blood phenylalanine levels. In patients with BH4 deficiency, sapropterin dihydrochloride is proposed to restore endogenous phenylalanine hydroxylase activity by providing an exogenous source of the missing cofactor.

Tetrahydrobiopterin is an unstable compound; at ambient temperature it is prone to autoxidation in the presence of molecular oxygen Tetrahydrobiopterin is also very hygroscopic. Therefore the development of stable oral composition comprising tetrahydrobiopterin that is prone to degradation at room temperature is a challenging task.

Thus, though researchers have developed compositions of sapropterin comprising stabilizers in variety of ratios, the stability of these compositons is low at room temperature or 40°C/75% relative humidity and need to be stored under refrigeration. Low stability of such tetrahydrobiopterin compositions is commercially undesirable and significant degradation due to improper storage could hinder therapy. Need therefore, exists for preparations of tetrahydrobiopterin that are more stable and retain desired amount of active over a longer time even when not refrigerated.

Further, the amount and type of stabilizer and other excipients present in the compositions of sapropterin determine the stability of the active and compositions thereof. Too little or too much stabilizer can affect the stability of the compositions of sapropterin and an appropriate amount of stabilizer must therefore be present in these compositions.

The stable formulations of sapropterin dihydrochloride according to the present invention thus provide desired amount of active over the entire shelf life of the product.

The solubility and dissolution rate of sapropterin dihydrochloride directly influence its bioavailability. For this reason, it is quite important to increase the solubility and dissolution rate of sapropterin dihydrochloride. A desired dissolution profile of the pharmaceutical formulation is obtained.

### Detailed Description of the Invention

The main object of the present invention is to provide pharmaceutical formulations, as defined in claim 1, comprising sapropterin dihydrochloride and at least one antioxidant with desired stability and bioavailability.

The term "sapropterin dihydrochloride" as used throughout the specification refers to not only sapropterin dihydrochloride, but also its other pharmaceutically acceptable solvates, pharmaceutically acceptable hydrates, pharmaceutically acceptable enantiomers, or pharmaceutically acceptable polymorphs thereof.

The amount of sapropterin dihydrochloride is between 20.0-30.0% preferably 25.0-30.0% by weight of the pharmaceutical formulation.

The present invention is to provide a stable pharmaceutical formulation by appropriate ratio of sapropterin dihydrochloride to antioxidant.

According to the invention, the antioxidants are selected from alpha tocopherol(Vitamin E), quercetine, butylhydroxyanisole (BHA), butylhydroxytoluene (BHT), citric acid or mixtures thereof.

The amount of antioxidant is between 5.6-6.4% by weight of the pharmaceutical formulation.

According to the present invention, the weight ratio of sapropterin dihydrochloride to antioxidant is between 3.0-6.0(w/w), preferably 3.5-5.5(w/w), more preferably 4.0-5.4 (w/w)
In a preferred embodiment according to the present invention, said pharmaceutical formulation further comprises at least one pharmaceutically acceptable excipient which is selected from fillers, binders, disintegrants, lubricants, sweeteners, coloring agents, buffering agent or mixtures thereof.

Suitable fillers are selected from the group comprising microcrystalline cellulose, mannitol, spray-dried mannitol, lactose, lactose monohydrate, lactose anhydrous, starch, dextrose, sucrose, fructose, maltose, sorbitol, xylitol, inositol, kaolin, inorganic salts, calcium salts, polysaccharides, dibasic calcium phosphate, sodium chloride, dextrates, lactitol, maltodextrin, sucrose-maltodextrin mixture, trehalose, sodium carbonate, sodium bicarbonate, calcium carbonate or mixtures thereof.

According to one embodiment of the present invention, fillers are used to achieve the desired dissolution profile, especially, when the amount of fillers is between 20.0% and 90.0%, preferably between 25.0% and 75.0%, so the pharmaceutical formulation shows desired dissolution profile.

According to one embodiment of the present invention, the amount of fillers is between 20.0% and 90.0%, 25.0% and 80.0%, 25.0% and 70.0%, 25.0% and 60.0%, 25.0% and 50.0%, 25.0% and 40.0%, 65.0% and 75.0%, 22.0% and 35.0% by weight of the pharmaceutical formulation. These ratios help to provide the desired dissolution of the formulation.

According to one embodiment of the present invention, the filler is lactose or sorbitol or microcrystalline cellulose or mixtures thereof.

Suitable binders are selected from the group comprising polyvinylpyrrolidone, polyethylene glycol, polyvinyl alcohol, starch, pregelatinized starch, glucose, glucose syrup, natural gums, sucrose, sodium alginate, lactose anhydrous, hydroxypropyl methyl cellulose, hydroxypropyl cellulose, carboxy methyl cellulose, methyl cellulose, gelatin, carrageenan, guar gum, carbomer, polymethacrylates, methacrylate polymers, collagenselatin, agar, alginate, alginic acid, xanthan gum, hyaluronic acid, pectin, polysaccharides, carbomer, poloxamer, polyacrylamide, aluminium hydroxide, laponite, bentonite, polyoxyethylene-alkyl ether, polydextrose, polyethylene oxide or mixtures thereof.

According to one embodiment of the present invention, the amount of binder is between 1.0% and 50.0%, 5.0% and 40.0%, preferably 10.0% and 30.0%, 15.0% and 25.0% by weight of the pharmaceutical formulation.

According to one embodiment of the present invention, the binder is lactose anhydrous.

Suitable disintegrants are selected from the group comprising polyvinyl pyrrolidone (crospovidone), crospovidone CL-Superfine (kollidon CL-Superfine), povidone, crosslinked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate or mixtures thereof.

According to one embodiment of the present invention, the amount of disintegrant is between 2.0% and 15.0%, 2.0% and 10.0%, 2.0% and 8.0% by weight of the pharmaceutical formulation.

According to one embodiment of the present invention, the disintegrant is sodium starch glycolate.

Suitable lubricants are selected from the group comprising from magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

Suitable coloring agents are selected from the group comprising curcumin, ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

Suitable sweeteners are selected from the group comprising potassium acesulfame, sodium saccharinate, neohesperidine dihydrochalcone, monoammonium glycyrrhizinate, sucralose, saccharin, sucrose, glucose, lactose, fructose, mannitol, xylitol, erythritol or mixtures thereof.

Suitable buffering agents which is selected from the group comprising glycine, sodium carbonate, alkali metal citrate, sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide or mixtures thereof, and preferably citric acid, fumaric acid, ascorbic acid, sodium dihydrogen phosphate, glycine , glutamic acid and mixtures thereof.

According to one embodiment of the present invention, the pharmaceutical formulation is in the form of soluble tablets, capsules, strips, powders, pastilles, sachets, effervescent compositions, pills, coated bead systems, granules, microspheres, dragees, films, orally administrable films, solids. Preferably, the pharmaceutical formulation is in the form of soluble tablets or sachets.

### Example 1: Sapropterin dihydrochloride and at least one antioxidant processed with dry granulation/direct compression (Soluble tablet production method)

| **Ingredients** | **Amount (%)** |
|---|---|
| Sapropterin dihydrochloride | 20.0-30.0% |
| Sodium starch glycolate | 2.0-15.0% |
| Monoammonium glycyrrhizinate | 0.5-10.0% |
| Lactose anhydrous | 1.0-50.0% |
| Microcrystalline cellulose | 20.0-90.0% |
| Antioxidant* | 5.6-7.5% |
| Curcumin | 1.0-4.0% |
| Magnesium Stearate | 0.25-5.0% |
| **TOTAL** | **100** |

### Process (Dry granulation/Direct compression)

a. Sieving Sapropterin dihydrochloride, Sodium starch glycolate, Monoammonium glycyrrhizinate, Lactose anhydrous, Microcrystalline cellulose, Antioxidant* and curcumin and mixing to obtain a homogenous mixture. The mixture is granulated.
b. Adding magnesium stearate to this mixture and mixing them.
c. Compressing this mixture into tablets.
   * Butylhydroxyanisole, Butylhydroxytoluene, vitamin E, Quercetine or Citric acid can be used as an antioxidant.

### Example 2: Sapropterin dihydrochloride and at least one antioxidant processed with dry granulation/direct compression (Soluble tablet production method)

| **Ingredients** | **Amount (%)** |
|---|---|
| Sapropterin dihydrochloride | 25.0-30.0% |
| Sodium starch glycolate | 2.0-10.0% |
| Monoammonium glycyrrhizinate | 0.5-10.0% |
| Lactose anhydrous | 5.0-40.0% |
| Microcrystalline cellulose | 20.0-80.0% |
| Antioxidant* | 5.6-6.4% |
| Curcumin | 1.0-4.0% |
| Magnesium Stearate | 0.25-5.0% |
| **TOTAL** | **100** |

### Process (Dry granulation/Direct compression)

a. Sieving Sapropterin dihydrochloride, Sodium starch glycolate, Monoammonium glycyrrhizinate, Lactose anhydrous, Microcrystalline cellulose, Antioxidant* and curcumin and mixing to obtain a homogenous mixture. The mixture is granulated.
b. Adding magnesium stearate to this mixture and mixing them.
c. Compressing this mixture into tablets.
   * Butylhydroxyanisole, Butylhydroxytoluene, vitamin E, Quercetine or Citric acid can be used as an antioxidant.

### Example 3: Sapropterin dihydrochloride and at least one antioxidant processed with dry granulation/direct compression (Soluble tablet production method)

| **Ingredients** | **Amount (%)** |
|---|---|
| Sapropterin dihydrochloride | 25.0-30.0% |
| Sodium starch glycolate | 2.0-8.0% |
| Monoammonium glycyrrhizinate | 0.5-10.0% |
| Lactose anhydrous | 15.0-25.0% |
| Microcrystalline cellulose | 20.0-30.0% |
| Antioxidant* | 5.6-6.4% |
| Curcumin | 1.0-4.0% |
| Magnesium Stearate | 0.25-5.0% |
| **TOTAL** | **100** |

### Process (Dry granulation/Direct compression)

a. Sieving Sapropterin dihydrochloride, Sodium starch glycolate, Monoammonium glycyrrhizinate, Lactose anhydrous, Microcrystalline cellulose, Antioxidant* and curcumin and mixing to obtain a homogenous mixture. The mixture is granulated.
b. Adding magnesium stearate to this mixture and mixing them.
c. Compressing this mixture into tablets.
   * Butylhydroxyanisole, Butylhydroxytoluene, vitamin E, Quercetine or Citric acid can be used as antioxidant.

### Example 4: Sapropterin dihydrochloride and at least one antioxidant processed with dry granulation/direct compression (Soluble tablet production method)

| **Ingredients** | **Amount (%)** |
|---|---|
| Sapropterin dihydrochloride | 28.0% |
| Sodium starch glycolate | 8.0% |
| Monoammonium glycyrrhizinate | 5.0% |
| Lactose anhydrous | 20.0% |
| Microcrystalline cellulose | 28.5% |
| Antioxidant* | 6.0% |
| Curcumin | 2.0% |
| Magnesium Stearate | 2.5% |
| **TOTAL** | **100** |

### Process (Dry granulation/Direct compression)

a. Sieving Sapropterin dihydrochloride, Sodium starch glycolate, Monoammonium glycyrrhizinate, Lactose anhydrous, Microcrystalline cellulose, Antioxidant* and curcumin and mixing to obtain a homogenous mixture. The mixture is granulated.
b. Adding magnesium stearate to this mixture and mixing them.
c. Compressing this mixture into tablets
   * Butylhydroxyanisole, Butylhydroxytoluene, vitamin E, Quercetine or Citric acid can be used as antioxidant.

### Example 5: Sapropterin dihydrochloride and at least one antioxidant processed with mixture (Powder for oral solution / sachet)

| **Ingredients** | **Amount (%)** |
|---|---|
| Sapropterin dihydrochloride | 20.0-30.0% |
| Sorbitol | 25.0-90.0% |
| Lactose | 25.0-90.0% |
| Antioxidant* | 5.6-6.4% |
| Sodium Citrate | 0.2-2.0% |
| **TOTAL** | **100** |

| | |
|---|---|
| Seiving sapropterine dihydrochloride and all excipients, then mixing to obtain homogenous powder mixture. Then the powder mixture packed in a sachet. * Butylhydroxyanisole, Butylhydroxytoluene, vitamin E, Quercetine or Citric acid can be used as antioxidant. | |

### Example 6: Sapropterin dihydrochloride and at least one antioxidant processed with mixture (Powder for oral solution / sachet)

| **Ingredients** | **Amount (%)** |
|---|---|
| Sapropterin dihydrochloride | 20.0% |
| Sorbitol | 40.0% |
| Lactose | 32.5% |
| Antioxidant* | 6.0% |
| Sodium Citrate | 1.5% |
| **TOTAL** | **100** |

| | |
|---|---|
| Seiving sapropterine dihydrochloride and all excipients, then mixing to obtain homogenous powder mixture. Then the powder mixture packed in a sachet. * Butylhydroxyanisole, Butylhydroxytoluene, vitamin E, Quercetine or Citric acid can be used as antioxidant. | |

## Claims

1. A pharmaceutical formulation comprising sapropterin dihydrochloride, and at least one antioxidant selected from quercetine, alpha tocopherol (vitamin E), butylhydroxyanisole, butylhydroxytoluene, citric acid or mixtures thereof wherein the weight ratio of sapropterin dihydrochloride to the antioxidant is between 3.0 - 6.0 (w/w) in total formulation and the amount of antioxidant is between 5.6-6.4% by weight of the total formulation.

2. The pharmaceutical formulation according to claim 1, wherein the weight ratio of sapropterin dihydrochloride to antioxidant is preferably between 4.0 - 5.4 (w/w) in the total formulation.

3. The pharmaceutical formulation according to any preceding claims, wherein the amount of the sapropterin dihydrochloride is 20-30% by weight, preferably 25-30% by weight of the total formulation.

4. The pharmaceutical formulation according to any preceding claims, further comprising at least one pharmaceutically acceptable excipient which is selected from fillers, binders, disintegrants, lubricants, sweeteners, coloring agents, buffering agent or mixtures thereof.

5. The pharmaceutical formulation according to claim 4, wherein fillers are selected from the group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dibasic calcium phosphate, sodium chloride, dextrates, lactitol, maltodextrin, sucrose-maltodextrin mixture, trehalose, sodium carbonate, sodium bicarbonate, calcium carbonate or mixtures thereof.

6. The pharmaceutical formulation according to claim 5, wherein the amount of fillers is between 20.0% and 90.0% by weight.

7. The pharmaceutical formulation according to any preceding claims, wherein the dosage form of the formulation is in the form of soluble tablets, capsules, strips, powders, pastilles, sachets, effervescent compositions, pills, coated bead systems, granules, microspheres, dragees, films or orally administrable films. Preferably, the pharmaceutical formulation is in the form of soluble tablets or sachets.

8. The pharmaceutical formulation according to claim 7, wherein the dosage form of the formulation is a soluble tablet.

9. The pharmaceutical formulation according to claim 7, wherein the dosage form of the formulation is a sachet.

10. The pharmaceutical formulation according to claim 8, soluble tablet consisting of;
25.0-30.0 % by weight of sapropterin dihydrochloride
2.0 -8.0 % by weight of sodium starch glycolate
0.5 -10.0% by weight of monoammonium glycyrrhizinate
15.0-25.0 % by weight of lactose anhydrous
20.0-30.0% by weight of microcrystalline cellulose
5.6-6.4 %by weight of quercetine, vitamin E, butylhydroxyanisole, butylhydroxytoluene or citric
acid
1.0-4.0 % by weight of curcumin
0.25-5.0% by weight of magnesium stearate.

11. The pharmaceutical formulation according to claim 9, sachet consisting of;
20.0-30.0 % by weight of sapropterin dihydrochloride
25.0 - 40.0 % by weight of sorbitol
25.0 - 32.5 % by weight of lactose
5.6 - 6.4 % by weight of quercetine, vitamin E, butylhydroxyanisole, butylhydroxytoluene or citric acid
0.2 - 2.0 % by weight of sodium Citrate.

## Patentansprüche

1. Eine pharmazeutische Formulierung, umfassend Sapropterindihydrochlorid und mindestens ein Antioxidans, ausgewählt aus Quercetin, Alpha-Tocopherol (Vitamin E), Butylhydroxyanisol, Butylhydroxytoluol, Citronensäure oder Mischungen davon, wobei das Gewichtsverhältnis von Sapropterindihydrochlorid zu dem Antioxidans in der Gesamtformulierung zwischen 3,0 und 6,0 (w/w) liegt und die Menge des Antioxidans zwischen 5,6 und 6,4 Gew.-% der Gesamtformulierung beträgt.

2. Die pharmazeutische Formulierung nach Anspruch 1, wobei das Gewichtsverhältnis von Sapropterindihydrochlorid zu Antioxidans in der Gesamtformulierung vorzugsweise zwischen 4,0 und 5,4 (w/w) liegt.

3. Die pharmazeutische Formulierung nach einem der vorhergehenden Ansprüche, wobei die Menge des Sapropterindihydrochlorids 20 bis 30 Gew.-%, vorzugsweise 25 bis 30 Gew.-% der Gesamtformulierung beträgt.

4. Die pharmazeutische Formulierung nach einem der vorhergehenden Ansprüche, die ferner mindestens einen pharmazeutisch verträglichen Hilfsstoff umfasst, der ausgewählt ist aus Füllstoffen, Bindemitteln, Sprengmitteln, Schmiermitteln, Süßungsmitteln, Farbstoffen, Puffermitteln oder Mischungen davon.

5. Die pharmazeutische Formulierung nach Anspruch 4, wobei die Füllstoffe ausgewählt sind aus der Gruppe umfassend mikrokristalline Cellulose, Mannitol, sprühgetrocknetes Mannitol, Lactose, Lactose-Monohydrat, Stärke, Dextrose, Saccharose, Fructose, Maltose, Sorbitol, Xylitol, Inositol, Kaolin, anorganische Salze, Calciumsalze, Polysaccharide, Dicalciumphosphat, Natriumchlorid, Dextrate, Lactitol, Maltodextrin, Saccharose-Maltodextrin-Mischung, Trehalose, Natriumcarbonat, Natriumbicarbonat, Calciumcarbonat oder Mischungen davon.

6. Die pharmazeutische Formulierung nach Anspruch 5, wobei die Menge der Füllstoffe zwischen 20,0 Gew.-% und 90,0 Gew.-% liegt.

7. Die pharmazeutische Formulierung nach einem der vorhergehenden Ansprüche, wobei die Darreichungsform der Formulierung in Form von löslichen Tabletten, Kapseln, Strips, Pulvern, Pastillen, Sachets, brausenden Zusammensetzungen, Pillen, beschichteten Kügelchen-Systemen (Beads), Granulaten, Mikrokugeln, Dragees, Filmen oder oral verabreichbaren Filmen vorliegt. Vorzugsweise liegt die pharmazeutische Formulierung in Form von löslichen Tabletten oder Sachets vor.

8. Die pharmazeutische Formulierung nach Anspruch 7, wobei die Darreichungsform der Formulierung eine lösliche Tablette ist.

9. Die pharmazeutische Formulierung nach Anspruch 7, wobei die Darreichungsform der Formulierung ein Sachet ist.

10. Die pharmazeutische Formulierung nach Anspruch 8, wobei die lösliche Tablette bestehend aus:
25,0-30,0 Gew.-% Sapropterindihydrochlorid
2,0-8,0 Gew.-% Natriumstärkeglycolat
0,5-10,0 Gew.-% Monoammoniumglycyrrhizinat
15,0-25,0 Gew.-% wasserfreier Lactose
20,0-30,0 Gew.-% mikrokristalliner Cellulose
5,6-6,4 Gew.-% Quercetin, Vitamin E, Butylhydroxyanisol, Butylhydroxytoluol oder Citronensäure
1,0-4,0 Gew.-% Curcumin
0,25-5,0 Gew.-% Magnesiumstearat.

11. Die pharmazeutische Formulierung nach Anspruch 9, wobei das Sachet bestehend aus:
20,0-30,0 Gew.-% Sapropterindihydrochlorid
25,0-40,0 Gew.-% Sorbitol
25,0-32,5 Gew.-% Lactose
5,6-6,4 Gew.-% Quercetin, Vitamin E, Butylhydroxyanisol, Butylhydroxytoluol oder Citronensäure
0,2-2,0 Gew.-% Natriumcitrat.

## Revendications

1. Formulation pharmaceutique comprenant du dichlorhydrate de saproptérine, et au moins un antioxydant choisi parmi la quercétine, l'alpha-tocophérol (vitamine E), le butylhydroxyanisole, le butylhydroxytoluène, l'acide citrique ou leurs mélanges, dans laquelle le rapport pondéral du dichlorhydrate de saproptérine à l'antioxydant est compris entre 3,0 - 6,0 (p/p) dans la formulation totale et la quantité d'antioxydant est comprise entre 5,6 - 6,4 % en poids de la formulation totale.

2. Formulation pharmaceutique selon la revendication 1, dans laquelle le rapport pondéral du dichlorhydrate de saproptérine à l'antioxydant est de préférence compris entre 4,0 - 5,4 (p/p) dans la formulation totale.

3. Formulation pharmaceutique selon l'une quelconque des revendications précédentes, dans laquelle la quantité de dichlorhydrate de saproptérine est de 20 - 30 % en poids, de préférence de 25 - 30 % en poids de la formulation totale.

4. Formulation pharmaceutique selon l'une quelconque des revendications précédentes, comprenant en outre au moins un excipient pharmaceutiquement acceptable qui est choisi parmi les charges, les liants, les désintégrants, les lubrifiants, les édulcorants, les agents colorants, les agents tampons ou leurs mélanges.

5. Formulation pharmaceutique selon la revendication 4, dans laquelle les charges sont choisies dans le groupe comprenant la cellulose microcristalline, le mannitol, le mannitol séché par atomisation, le lactose, le lactose monohydraté, l'amidon, le dextrose, le saccharose, le fructose, le maltose, le sorbitol, le xylitol, l'inositol, le kaolin, les sels inorganiques, les sels de calcium, les polysaccharides, le phosphate de calcium dibasique, le chlorure de sodium, les dextrates, le lactitol, la maltodextrine, un mélange saccharose-maltodextrine, le tréhalose, le carbonate de sodium, le bicarbonate de sodium, le carbonate de calcium ou leurs mélanges.

6. Formulation pharmaceutique selon la revendication 5, dans laquelle la quantité de charges est comprise entre 20,0 % et 90,0 % en poids.

7. Formulation pharmaceutique selon l'une quelconque des revendications précédentes, dans laquelle la forme posologique de la formulation se présente sous la forme de comprimés solubles, de gélules, de bandes, de poudres, de pastilles, de sachets, de compositions effervescentes, de pilules, de systèmes de billes enrobées, de granulés, de microsphères, de dragées, de films ou de films administrables par voie orale. De préférence, la formulation pharmaceutique se présente sous la forme de comprimés solubles ou de sachets.

8. Formulation pharmaceutique selon la revendication 7, dans laquelle la forme posologique de la formulation est un comprimé soluble.

9. Formulation pharmaceutique selon la revendication 7, dans laquelle la forme posologique de la formulation est un sachet.

10. Formulation pharmaceutique selon la revendication 8, sous forme de comprimé soluble constituée de :
25,0-30,0 % en poids de dichlorhydrate de saproptérine
2,0-8,0 % en poids de glycolate d'amidon sodique
0,5-10,0 % en poids de glycyrrhizinate de monoammonium
15,0-25,0 % en poids de lactose anhydre
20,0-30,0 % en poids de cellulose microcristalline
5,6-6,4 % en poids de quercétine, de vitamine E, de butylhydroxyanisole, de butylhydroxytoluène ou d'acide citrique
1,0-4,0 % en poids de curcumine
0,25-5,0 % en poids de stéarate de magnésium.

11. Formulation pharmaceutique selon la revendication 9, sous forme de sachet constituée de :
20,0-30,0 % en poids de dichlorhydrate de saproptérine
25,0-40,0 % en poids de sorbitol
25,0-32,5 % en poids de lactose
5,6-6,4 % en poids de quercétine, de vitamine E, de butylhydroxyanisole, de butylhydroxytoluène ou d'acide citrique
0,2-2,0 % en poids de citrate de sodium.
